# FASCICULE DE BREVET EUROPEEN

(11) **EP 1 379 217 B1**
(45) Date de publication et mention de la délivrance du brevet: **29.12.2004**
(21) Numéro de dépôt: 02730115.9
(22) Date de dépôt: 06.04.2002
(51) Int. Cl.: A61K 7/48

(54) **PRODUIT COSMETIQUE POUR L'ENTRETIEN DE L'EPIDERME A BASE D'UN MELANGE TERNAIRE**
KOSMETISCHES PRODUKT FÜR DIE PFLEGE DER EPIDERMIS AUF BASIS EINER DREIKOMPONENTEN-MISCHUNG
COSMETIC PRODUCT FOR SKIN CARE BASED ON A THREE-COMPONENTS MIXTURE

(30) Priorité: 19.04.2001 MC 2464
(43) Date de publication de la demande: 14.01.2004
(73) Titulaire: Eisenberg, José, 98002 Monaco (MC)
(72) Inventeur: Eisenberg, José, 98002 Monaco (MC)
(74) Mandataire: Hautier, Jean-Louis
(86) Numéro de dépôt international: PCT/EP2002/003826
(87) Numéro de publication internationale: WO 2002/085323

(56) Documents cités:
- CH-A- 686 999
- CH-A- 687 000
- FR-A- 2 697 750
- DATABASE WPI Section Ch, Week 199913 Derwent Publications Ltd., London, GB; Class D16, AN 1993-217136 XP002223651 & KR 9 604 015 B (SOPHIL LTD), 25 mars 1996 (1996-03-25)

## Description

L'invention a pour objet un produit cosmétique pour l'entretien de l'épiderme à base d'un complexe trio moléculaire.

L'état de la technique peut être défini par les brevets suivants qui décrivent l'action des cytokines.

Le brevet EP 0 826 367 décrit l'action d'un extrait de bertholletia pour stimuler la synthèse du collagène.

Cet art antérieur concerne de nouvelles utilisations d'un extrait de Bertholletia dans le domaine cosmétique ou pharmaceutique, notamment dermatologique et pour la préparation de milieux de culture de cellules.

Elle concerne plus particulièrement les utilisations dans le domaine cosmétique où pharmaceutique où l'on cherche à stimuler la synthèse du collagène VII et/ou à favoriser l'incorporation de la vitamine C dans les cellules de la peau, en particulier dans les fibroblastes.

Elle concerne également de nouveaux milieux de culture de cellules contenant un extrait de bertholletia.

Le brevet FR-A-2743817 concerne un modèle de peau reconstruite, caractérisé par le fait qu'il comprend un équivalent d'épiderme sur un support, ledit équivalent d'épiderme comprenant au moins des kératinocytes et des cellules de Langerhans et/ou des précurseurs des cellules de Langerhans ainsi que le procédé de préparation dudit équivalent de peau.

FR-A-2743817 concerne également un équivalent d'épiderme, caractérisé par le fait qu'il comprend au moins des kératinocytes et des cellules de Langerhans et/ou des précurseurs des cellules de Langerhans et son procédé de préparation.

Le produit cosmétique selon l'invention a pour objet d'utiliser des principes actifs qui, en combinaison, réactivent les caractéristiques de la peau pour que celle-ci reste souple, tonique, et élastique.

L'invention réside dans le fait de combiner trois molécules, chacune ayant une fonction propre, des enzymes, des cytokines, des biostimulines.

La combinaison de ces trois fonctions apportant un résultat spectaculaire, notamment visuel sur la peau.

Les enzymes ont un effet kératolitique sur la peau.

Les cytokines ont pour fonction de stimuler l'activité cellulaire, dont le renouvellement cellulaire.

Les biostimulines ont pour fonction de favoriser l'oxygénation des cellules, source d'énergie vitale.

La synthèse du collagène et de l'élastine garantissant la tonicité et l'élasticité de la peau.

Les enzymes sont des Bacillus Subtilis.

Les cytokines sont des polypeptides extraits du lait Lactis Proteïnum.

Les biostimulines sont extraits de tissus frais de jeunes pousses de hêtre, Fagus Silvatica.

Les principes actifs utilisés en combinaison pour agir entre eux sont :
- Des enzymes à effet kératolitique. Ces molécules biotechnologiques ont pour rôle de digérer et d'éliminer les cornéocytes desquamants.

Elles favorisent une parfaite assimilation des principes actifs et garantissent une meilleure qualité du manteau cellulaire.
- Des cytokines molécules clés de l'autocontrôle cellulaire. Les cytokines sont des messagers de nature peptidique émis par certaines cellules (globules blancs, cellules du système immunitaire). Elles agissent sur les récepteurs d'autres cellules pour induire de nombreuses réactions vitales, dont le renouvellement cellulaire.

Les cytokines sont extraites du lait. Ce sont des polypeptides (groupes d'acides aminés) sélectionnés, bio-actifs avec une activité biologique régulée et vérifiable.
- Des biostimulines oxygénant les cellules et favorisant la synthèse protéique. Les biostimulines de tissus frais de jeunes pousses de hêtre, dont la composition est très proche des protéines de notre tissu cutané, favorisent :
   - L'oxygénation des cellules, source d'énergie vitale
   - La synthèse du collagène et de l'élastine garantissant la tonicité, l'élasticité.

Les enzymes à effet kératolitique. Par fermentation d'un micro-organisme : le Bacillus Subtilis, on extrait un enzyme (une protéase) dont la capacité réside dans l'élimination des cornéocytes desquamants. L'action enzymatique naturelle permet d'effectuer en douceur le nettoyage de la peau et ainsi accélérer son renouvellement. Elle est étalée dans le temps, en fonction des besoins. La kératine morte des cornéocytes est digérée, la cohésion cellulaire des cellules desquamantes est brisée, la peau peut se renouveler plus rapidement.

Les biostimulines sont obtenues à partir de jeunes tissus frais de hêtre (Fagus Silvatica L). A une période précise et courte de l'année (période de la cueillette), ces tissus sont concentrés en biostimulines végétales.

Les acides aminés de ces biostimulines sont similaires aux protéines structurales du tissu cutané (collagène). Elles sont également riches en molécules essentielles (sels minéraux, peptides) et amino acides (Proline, Tryptophane, Alanine) qui jouent un rôle primordial dans le métabolisme cellulaire.

Les biostimulines utilisées à 20 % augmentent la consommation d'oxygène de 71 % (8830 unités oxygraphiques) donc une oxygraphique très importante.

Les biostimulines sont non cytotoxiques à l'égard des fibroblastes et des kératinocytes, kératinocytes qui bénéficieront les premiers de l'action du produit au niveau cutané.

Les biostimulines peuvent avoir une action sur la synthèse de protéines essentielles produites par les kératinocytes comme la Kératine, la filaggrine, l'involucrine, les protéines de transport, les protéines immunitaires...

Une augmentation de 42 % de la synthèse protéique est provoquée par l'addition de 0,4 % de biostimulines.

Les biostimulines ne modifient pas la morphologie des cellules avec lesquelles elles sont en contact et ne provoquent aucune prolifération cellulaire.

Le vieillissement est un processus dynamique qui agit sur tout le corps à différents niveaux.

Outre le processus de vieillissement naturel, génétiquement programmé, s'ajoutent des perturbations extérieures qui provoquent une modification des relations structure/fonction dans la peau : il faut donc faire une distinction entre le vieillissement biologique et le vieillissement accéléré (prématuré).

Les relations structure/fonction s'établissent dans la peau entre la matrice extracellulaire et les cellules.

La matrice extracellulaire est un compartiment hautement organisé et complexe, constitué par différents types de collagène, l'élastine, les protéoglycanes, la laminine et la fibronectine.

Une cellule doit maintenir une information moléculaire constante sur son compartiment extracellulaire afin de s'adapter aux variations de son environnement.

Ces informations sont transmises via les récepteurs cellulaires et les intégrines (molécules d'adhésion) qui permettent de coordonner l'activité de la cellule à celle du tissu dont elle relève (fonction).

La connaissance des mécanismes de régulation et de leurs facteurs de contrôle s'est largement étendue.

Le terme général de Cytokine recouvre un certain nombre de polypeptides définis. Ceux-ci représentent le système d'information des cellules à la fois en tant que molécules-signal et en tant que facteurs de croissance ou d'inhibition, et ils assument une tâche déterminée. Ces molécules interagissent avec les cellules grâce à des récepteurs spécifiques.

Les cytokines contrôlent le bon maintient et le rétablissement de l'homéostasie, l'équilibre fonctionnel entre les cellules et leur environnement extracellulaire.

Les cytokines sont les molécules-clé du contrôle cellulaire. Ces composants interagissent de façon coordonnée afin de restaurer et de maintenir l'intégrité cutanée.

Les cytokines, le lait est en soi un aliment presque complet pour maintenir la vie, mais le lait est toutefois beaucoup plus qu'une source alimentaire. On a pu prouver que le lait contient un vaste assortiment de polypeptides actifs biologiquement et capables de stimuler et de réguler, mais aussi d'inhiber un certain nombre de fonctions cellulaires.

Un nombre croissant de preuves permettent d'affirmer que les peptides du lait possèdent de réelles fonctions physiologiques (Pour avoir un aperçu de cette question, cf Britton and Kastin, Am. J. Med. Sci. 1991, 301 (2), 124-132).

Le rôle de certains de ces peptides du lait a été établi : ils agissent comme les médiateurs naturels des processus cellulaires chargés du maintien de la peau en bon état (cf. Pittelkow, Advences in Dermatology vol. 7.55-81 (1991)).

De tels polypeptides font partie des facteurs de croissance peptidiques et se rangent dans la classe générale des Cytokines.

On a pu isoler dans le lait la plupart des Cytokines les plus importantes :
- Facteur de Croissance Epidermique (EGF) (Carpenter, 1980 ; Beardmore, Richards, 1983; Pétrides et al., 1985; Yagi et al., 1986)
- Facteur de Croissance de Transformation α (TGF - α) (Sweibel et al., 1986 ; Okada et al., 1991)
- Facteur de Croissance de Transformation β - 1 et β - 2 (TGF - β 1, 2) (Cox, Bürk, 1991 ; Jin et al., 1991)
- Facteur de Stimulation des Colonies (CSF) (Sinha, Yunis, 1983)
- Facteur de Croissance pseudo-insuline I et II (IGF) (Suikkari, 1989 ; Nagashima et al., 1990 ; Donovan et al., 1991)
- Interieukine - 1 et 2 (IL-1, IL-2) (Söder, 1987 ; Britton et al., 1991)
- Facteur de Croissance des Nerfs (NGF) (Britton et al., 1991 )

Les cytokines présentent dans le lait sont sous forme désactivée. Par un procédé en plusieurs étapes il a été rendu possible en partant du lait de leur redonner leur forme biologiquement active.

Ce complexe contient aussi la lactalbumine, le lactose, la lactoglobuline, la lactoferrine. Ces composants possèdent leur valeur propre et leur fonction en tant que substances cosmétiques, mais la fonction essentielle est avant tout à stabiliser les cytokines activées.

La présente invention concerne un produit cosmétique pour réactiver les caractéristiques de la peau pour que celle-ci reste souple, tonique, et élastique, les enzymes ont un effet kératolitique sur la peau ; les cytokines ont pour fonction de stimuler l'activité cellulaire, dont le renouvellement cellulaire; les biostimulines ont pour fonction de favoriser l'oxygénation des cellules, source d'énergie vitale.

Il comporte une combinaison de trois principes actifs :
- des enzymes (Protease) obtenues et extraits de la fermentation des Bacillus Subtillis, des cytokines, des biostimulines,
- que les cytokines sont des polypeptides extraits du lait Lactis Proteïnum.
- que les biostimulines sont extraits de tissus frais de jeunes pousses de hêtre, Fagus Silvatica.

## Revendications

1. Produit cosmétique pour réactiver les caractéristiques de la peau pour que celle-ci reste souple, tonique, et élastique, les enzymes ont un effet kératolitique sur la peau ; les cytokines ont pour fonction de stimuler l'activité cellulaire, dont le renouvellement celluiaire ; les biostimulines ont pour fonction de favoriser l'oxygénation des cellules, source d'énergie vitale.
**caractérisé par le fait,**
**qu'**il comporte une combinaison de trois principes actifs :
- des enzymes (Protease) obtenues et extraits de la fermentation des Bacillus Subtillis, des cytokines, des biostimulines,
- **que** les cytokines sont des polypeptides extraits du lait Lactis Proteïnum.
- **que** les biostimulines sont extraits de tissus frais de jeunes pousses de hêtre, Fagus Silvatica.

## Claims

1. Cosmetic product for reviving skin characteristics so that it stays flexible, tonic, and elastic, the enzymes have a keratolitic effect on the skin; the cytokines have the function to stimulate cellular activity, including cellular renewal ; the biostimulines have the function to encourage the oxygenation of the cells, a source of vital energy.
**characterized in that**,
it comprises a combination of three active ingredients:
- enzymes (Protease) obtained and extracted from the fermentation of bacillus subtillis, cytokines, biostimulines,
- the cytokines are the polypeptides extracted from the milk lactis proteinum.
- the biostimulines are extracted from the new tissue of young beech shoots, fagus silvatica.

## Patentansprüche

1. Kosmetikprodukt zur Reaktivierung der Hautmerkmale, damit diese weich, straff und elastisch bleibt, wobei Enzyme eine keratolytische Wirkung auf die Haut haben; Zytokine die Funktion haben, die Zellaktivität und damit die Zellemeuerung zu stimulieren, Biostimulatoren die Funktion haben, die Versorgung der Zellen mit Sauerstoff als vitaler Energiequelle zu fördern, **gekennzeichnet dadurch,**
**dass** es eine Kombination aus drei Wirkstoffen enthält:
• Enzymen (Proteasen), die durch Extraktion aus der Fermentation des Bacillus Subtillis gewonnen wurden, Zytokine und Biostimulatoren,
• wobei die Zytokine aus Lactis Proteneinum-Milch extrahierte Polypeptide sind,
• und die Biostimulatoren aus frischem Gewebe junger Buchentriebe (Fagus silvatica) gewonnen werden.
